# EUROPEAN PATENT SPECIFICATION

(11) **EP 1 393 691 B1**
(45) Date of publication and mention of the grant of the patent: **29.12.2004**
(21) Application number: 03077261.0
(22) Date of filing: 18.07.2003
(51) Int. Cl.: A61B 19/02

(54) **Surgical console**
Chirurgische Console
Console chirurgicale

(30) Priority: 26.08.2002 US 227919
(43) Date of publication of application: 03.03.2004
(73) Proprietor: Alcon Inc., 6331 Hunenberg (CH)
(72) Inventor: Olivera, Argelio M., Los Alamitos, CA 91720 (US)
(74) Representative: Moore, Barry

(56) References cited:
- EP-A- 0 420 624
- EP-A- 0 437 370
- DE-A- 2 916 448
- US-A- 5 415 287
- US-A- 6 086 598

## Description

### Background of the Invention

This invention relates generally to the field of surgical consoles and, more particularly, to microsurgical consoles.

During modem surgery, particularly ophthalmic surgery, the surgeon uses a variety of pneumatic and electronically driven microsurgical handpieces. The handpieces are operated by a microprocessor-driven surgical console that receives inputs from the surgeon or an assistant by a variety of peripheral devices, such as footswitches and infrared remote control devices. These peripheral devices, particularly footswitches, must be moved when the console is moved and can be heavy and bulky. While some prior art consoles provided a hook or some other handing mechanism on the back of the console, these exposed hooks did not protect the footswitch or the footswitch cabling. Another device, described in U.S. Patent Publication No. 20010035702 (Murphy, et al.), the entire contents of which being incorporated herein by reference, discloses a footswitch storage drawer. The connections for the footswitch, however, are external to the drawer, requiring the footswitch be disconnected from the console during storage.

Accordingly, a need continues to exist for a surgical console that provides a safe and convenient device for storing and transporting peripheral devices that does not require the peripheral device to be disconnected during storage, and reconnected during use.

### Brief Summary of the Invention

The present invention improves upon the prior art surgical consoles by providing a surgical console containing a protective drawer sized and shaped to receive a footswitch or other peripheral device. The electrical/communications connection between the peripheral device and the console is contained internally to the console. The drawer protects the peripheral device and any cabling associated with the peripheral device and provides a convenient and safe way of moving the peripheral device along with the console. The internal electrical/communications connection allows the device to be stored in the drawer without disconnecting the device from the console.

US 6,086,598 discloses a typical surgical console according to the preamble of claim 1.

Accordingly, one objective of the present invention is to provide a surgical console having a safe and convenient mechanism for moving peripheral devices along with the surgical console.

Another objective of the present invention is to provide a surgical console having a protective drawer sized and shaped to receive a footswitch or other peripheral device.

These and other advantages and objectives of the present invention will become apparent from the detailed description and claims that follow.

### Brief Description of the Drawings

FIG. 1 is a perspective view of the surgical console and peripheral device of the present invention showing the drawer in the closed position.
FIG. 2 is a perspective view of the surgical console and peripheral device of the present invention showing the drawer in the open position.
FIG. 3 is a perspective view of the surgical console and peripheral device of the present invention showing the drawer in the open position and the peripheral device stored within the drawer.

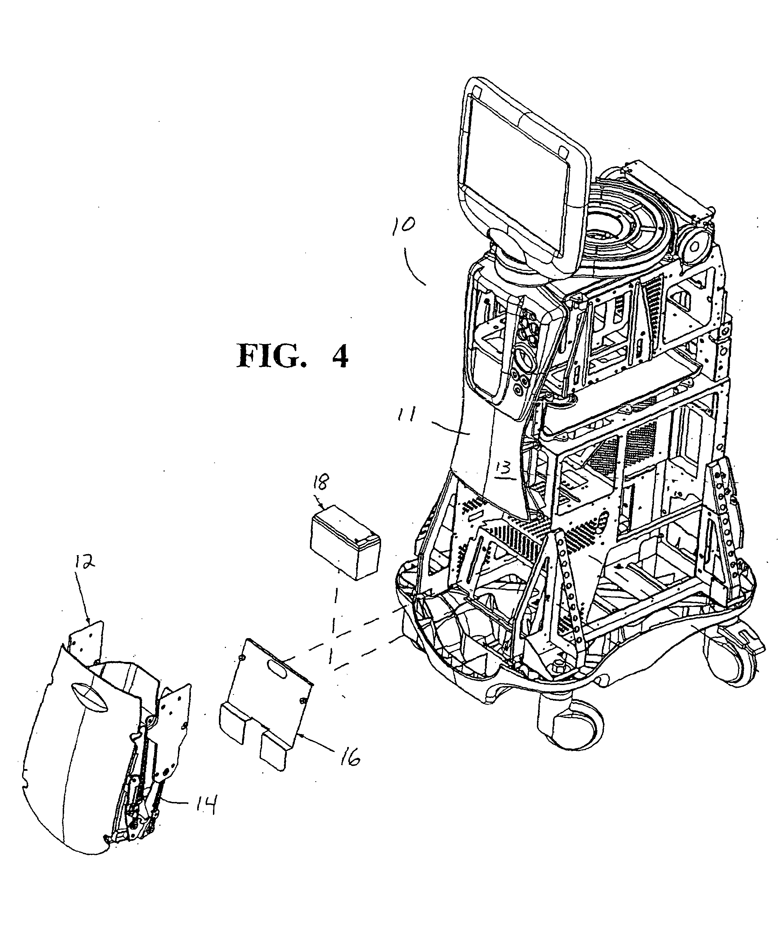

FIG. 4 is an exploded assembly view of the surgical console of the present invention.

### Detailed Description of the Invention

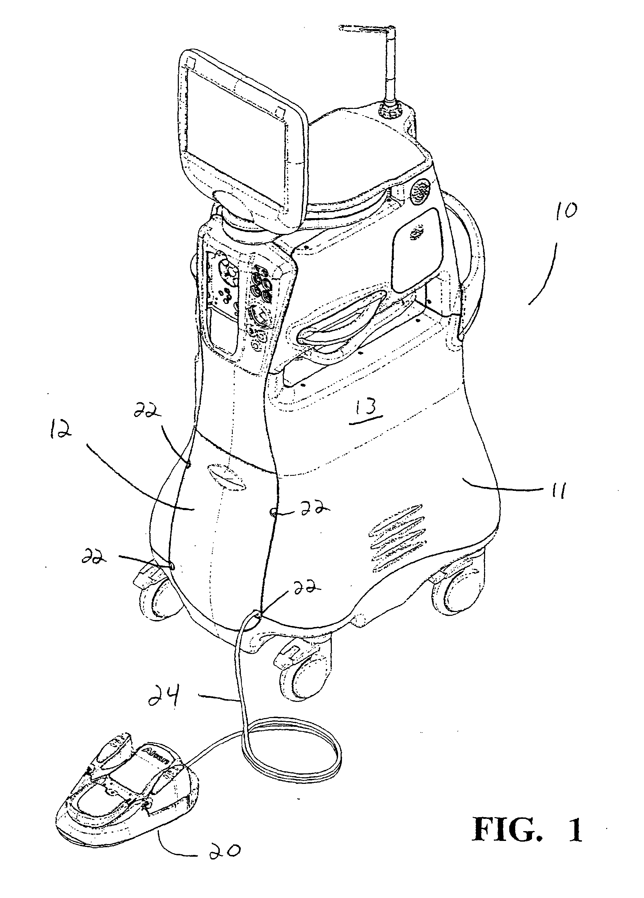

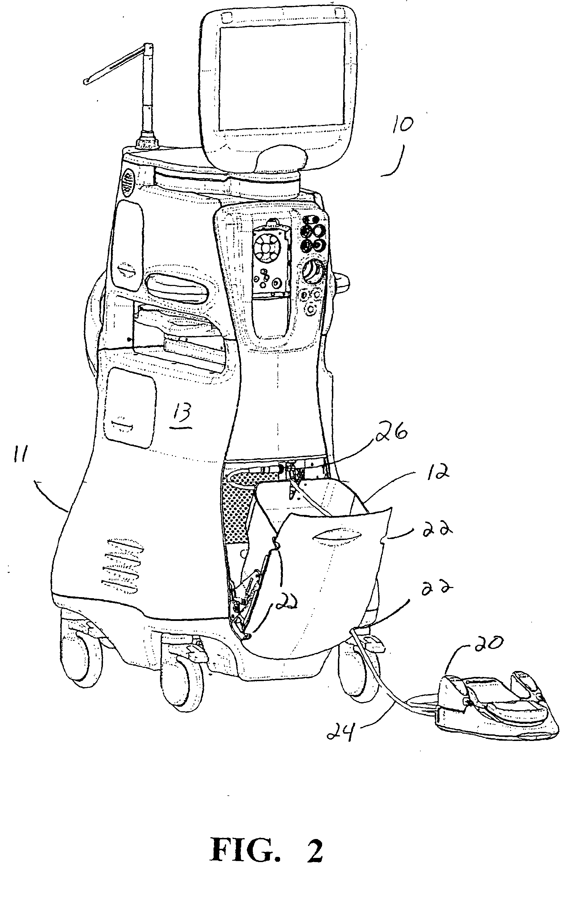

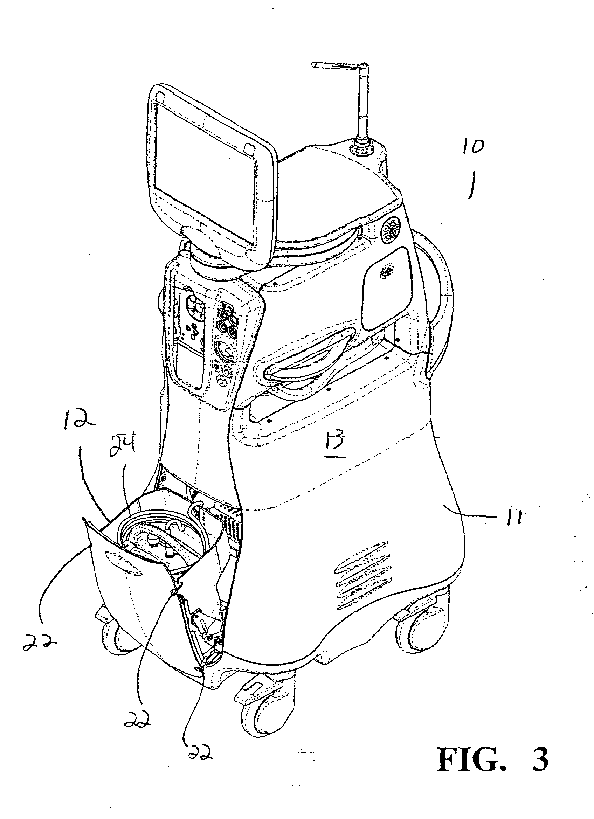

Surgical console 10 of the present invention may be any suitable console, such as the INFINITI™ surgical system console, as seen in FIGS. 1 and 2, commercially available from Alcon Laboratories, Inc., Fort Worth, Texas. Exterior 11 of body 13 of console 10 contains drawer 12 that is received on the front of console 10. Drawer 12 may be attached to body 13 by any suitable, hinged attachment device, such as four bar linkage 14. As best seen in FIG. 2, removal of drawer 12 from console 10 may provide access to removable panel 16. Removing panel 16 may provide for service access to the interior of console 10, for example, access to battery 18. Drawer 12 may be of any size or shape, but is preferably sized and shaped to receive a peripheral device, such as footswitch 20 and associated cabling, which are normally external to body 13. As best seen in FIG. 1, drawer 12 may contain a plurality of notches 22 that allows cable 24 associated with device 20 to exit drawer 12 when drawer 12 is closed. While a plurality of notches 22 are shown, and such plurality allows the cable to exit drawer 12 in a variety of positions, one skilled in the art will recognize that only one notch 22 is required. Cable 24 contains electrical wiring so as to allow device 20 to communicate with console 10 and receive power from console 10. As best seen in FIGS. 2 and 3, cable 24 connects on one end to device 20, and on the other end to console 10 through connection 26 internal to console 10. Such a construct allows device 20 to be stored in drawer 12 without disconnecting cable 24 from console 10.

This description is given for purposes of illustration and explanation. It will be apparent to those skilled in the relevant art that modifications may be made to the invention as herein described without departing from the scope of the claims.

## Claims

1. A surgical console (10), comprising:
a) a body (13) having an exterior (11);
b) a drawer (12) opening from the exterior of the body;
c) a peripheral device (20) connected to the console by a cable (24) through a connection internal to the body; **characterised in that** the internal connection is situated behind said drawer (12); said drawer (12) is sized for receiving said peripheral device (20), and containes at least one notch,
wherein the notch is sized and shaped to allow the cable to be connected to the body through the drawer when the drawer is in a closed position.

2. The console of claim 1 further wherein the peripheral device (20) comprising a footswitch.

3. The console of claim 2 wherein the drawer (12) is sized and shaped to receive the footswitch (20).

4. The console of claim 1 further comprising a removable panel (16) wherein removal of the drawer (12) provides access to the removable panel.

5. The console of claim 1 wherein the drawer (12) is hingedly attached to the body by a four bar linkage (14).

## Patentansprüche

1. Chirurgische Konsole (10), die folgendes umfaßt:
a) einen Körper (13) mit einer Außenseite (11),
b) einen Einschub (12), der sich aus der Außenseite des Körpers öffnet,
c) eine Peripherieeinrichtung (20), die mit der Konsole über ein Kabel (24) über eine Verbindung innerhalb des Körpers verbunden ist, **dadurch gekennzeichnet, daß** die innere Verbindung hinter dem Einschub (12) untergebracht ist, der Einschub (12) dazu bemessen ist, die Peripherieeinrichtung (20) aufzunehmen und wenigstens eine Nut aufweist, wobei die Nute so bemessen und geformt ist, um zu ermöglichen, daß das Kabel mit dem Körper durch den Einschub verbunden ist, wenn der Einschub sich in einer geschlossenen Position befindet.

2. Konsole nach Anspruch 1, bei der weiterhin die Peripherieeinrichtung (20) einen Fußschalter umfaßt.

3. Konsole nach Anspruch 2, bei dem der Einschub (12) so bemessen und geformt ist, um den Fußschalter (20) aufzunehmen.

4. Konsole nach Anspruch 1, der weiterhin ein entfembares Panel (16) umfaßt, wobei ein Entfernen des Einschubs (12) einen Zugang zu dem entfernbaren Panel liefert.

5. Konsole nach Anspruch 1, bei der der Einschub (12) klappbar an dem Körper über vier Gestängeverbindungen (14) angebracht ist.

## Revendications

1. Console chirurgicale (10), comprenant :
a) un corps (13) présentant un carrossage (11) ;
b) un tiroir (12) s'ouvrant à partir du carrossage du corps ;
c) un dispositif périphérique (20) connecté à la console par un câble (24) par l'intermédiaire d'une connexion à l'intérieur du corps ;
**caractérisée en ce que** la connexion intérieure est située derrière ledit tiroir (12) ; ledit tiroir (12) est dimensionné pour recevoir ledit dispositif périphérique (20), et comprend au moins encoche,
dans laquelle l'encoche est dimensionnée et formée afin de permettre au câble d'être connecté au corps au travers du tiroir lorsque le tiroir est en une position fermée.

2. Console selon la revendication 1, dans laquelle le dispositif périphérique (20) comprend en outre un interrupteur au pied.

3. Console selon la revendication 2, dans laquelle le tiroir (12) est dimensionné et conformé afin de recevoir l'interrupteur au pied (20).

4. Console selon la revendication 1, comprenant en outre un panneau amovible (16), dans laquelle enlever le tiroir (12) procure un accès au panneau amovible.

5. Console selon la revendication 1, dans laquelle le tiroir (12) est solidarisé par charnière au corps par une liaison à quatre barres (14).
